(19) Europäisches Patentamt
European Patent Office
Office européen des brevets

(11) **EP 3 263 011 A1**

(12) **EUROPEAN PATENT APPLICATION**
published in accordance with Art. 153(4) EPC

(43) Date of publication:
**03.01.2018 Bulletin 2018/01**

(51) Int Cl.:
***A61B 1/04*** (2006.01)     ***A61B 1/00*** (2006.01)

(21) Application number: **16728211.0**

(22) Date of filing: **23.02.2016**

(86) International application number:
**PCT/JP2016/055150**

(87) International publication number:
**WO 2016/136700 (01.09.2016 Gazette 2016/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.02.2015 JP 2015037540**

(71) Applicant: **Hoya Corporation**
**Shinjuku-ku**
**Tokyo 1608347 (JP)**

(72) Inventors:
• **IKEMOTO, Yousuke**
**Tokyo 160-8347 (JP)**
• **MINAKUCHI, Tadashi**
**Tokyo 160-8347 (JP)**
• **KOMORO, Atsushi**
**Tokyo 160-8347 (JP)**
• **TACHIBANA, Toshio**
**Tokyo 160-8347 (JP)**
• **OTA, Noriko**
**Tokyo 160-8347 (JP)**

(74) Representative: **Schaumburg und Partner**
**Patentanwälte mbB**
**Postfach 86 07 48**
**81634 München (DE)**

(54) **IMAGE PROCESSING DEVICE**

(57) An image processing apparatus is provided with an image data obtaining means configured to obtain color moving image data including multiple pieces of image data capturing biological tissues, a scene determining means configured to determine a photographic scene based on the color moving image data, a score calculating means configured to calculate a score indicative of seriousness of lesion of the biological tissues captured in the image represented by the image data for each pixel, based on the image data, and a marking means configured to apply marks indicative of a distribution of the scores on the image. The marking means is configured to execute a detailed marking process to apply the marks indicating the distribution of the scores in detail, and a simple marking process to apply the marks indicating the distribution of the scores in a manner simpler than the detailed marking process. The marking means executes one of the detailed marking process and the simple marking process in accordance with the result of determination of the photographic scene.

FIG. 4

**Description**

**Technical Field**

The present invention relates to an image processing apparatus configured to process an image of a biological tissue.

Generally, lesion parts of biological tissues exhibit a color different from that of normal parts. As the performance of color endoscopes has improved, it becomes possible the distinguish a lesion part of which color is only slightly different from the color of the normal parts. However, in order for an operator to accurately distinguish a lesion part from normal tissues by only a slight difference of the colors of the endoscopic image, the operator should have been trained by a skilled instructor for a long period. Further, it is not easy to distinguish the lesion parts by only a slight color difference for even a skilled operator, a careful operation is required.

It has been suggested, for example, in Japanese Patent Provisional Publication No. 2014-18332 (hereinafter, referred to as patent document 1), an endoscope apparatus in which, in order that lesion parts can be distinguished easily, with respect to endoscopic image data photographed under illumination of white light, whether an object is a lesion part of not based on color information, and a color mapping process to change colors of pixels of a portion which is judged to be the lesion part is executed.

**Summary of the Invention**

According to the endoscope apparatus of patent document 1, since the color mapping process is applied to all the pixels, a calculation amount necessary for the color mapping process is very large. Therefore, when a movement of an image is fast at a time of a screening inspection or the like, the color mapping process is slowed and the endoscopic image cannot follow the frame rate at which the endoscopic images area photographed. As a result, the color mapping is applied to another endoscopic image photographed after the endoscopic image for which the color mapping is to be applied, there has occurred a problem that a positional displacement occurs between the endoscopic image and the color mapping.

The present invention is made in view of the above circumstances and an object thereof is to provide an image processing apparatus capable of preventing positional displacement between marks applied to an endoscopic image to indicate lesion parts and the endoscopic image itself.

According to an embodiment of the present invention, there is provided an image processing apparatus, which has an image data obtaining means configured to obtain color moving image data including multiple pieces of image data capturing biological tissues, a scene determining means configured to determine a photographic scene based on the color moving image data, a score calculating means configured to calculate a score indicative of seriousness of lesion of the biological tissues captured in the image represented by the image data for each pixel, based on the image data, a marking means configured to apply marks indicative of a distribution of the scores on the image. The marking means is configured to execute a detailed marking process to apply the marks indicating the distribution of the scores in detail, and a simple marking process to apply the marks indicating the distribution of the scores in a manner simpler than the detailed marking process. The marking means executes one of the detailed marking process and the simple marking process in accordance with the result of determination of the photographic scene.

In the image processing apparatus described above, the scene determining means may determine a kind of image an image inspection photographed in the color moving image data.

In the image processing apparatuses described above, the scene determining means may determine whether the photographic scene is of a screening inspection or a thorough inspection, and the marking means may execute the simple marking process when the photographic scene is determined to be of the screening inspection, while the detailed marking process when the photographic scene is determined to be of the thorough inspection.

In the image processing apparatus described above, the scene determining means may be provided with a moving image analyzing means configured to analyze movement of the image.

In the image processing apparatus described above, the moving image analyzing means may be provided with a velocity field calculating means configured to calculate a velocity field based on continuous multiple pieces of image data, and may determine a kind of the image inspection based on a calculation result of the velocity field.

In the image processing apparatus described above, the moving image analyzing means may be provided with an image velocity calculating means configured to calculate a representative value of magnitude of velocity vectors of respective pixels constituting the velocity field and obtain the representative value as an image velocity.

In the image processing apparatus described above, the moving image analyzing means may be provided with an image velocity change rate calculating means configured to calculate an image velocity change rate which represents a rate of change of the image velocity per unit time.

In the image processing apparatus described above, the moving image analyzing means may be provided with a resolution lowering means configured to lower a resolution of the image data.

In the image processing apparatus described above, the scene determining means may be provided with a brightness image data generating means configured to generate brightness image data of which pixel values are brightness values of the image data.

In the image processing apparatus described above, the scene determining means may be provided with an image simplifying means configured to simplify a brightness image represented by the brightness image data.

In the image processing apparatus described above, the image simplifying means may be provided with a resolution lowering means configured to lower a resolution of the brightness image, a blurring means configured to apply a blurring processing to the brightness image of which resolution has been lowered, and a resolution increasing means configured to increase the resolution of the brightness image to which the blurring processing has been applied to an original resolution.

In the image processing apparatus described above, the image simplifying means may be provided with a gradation reducing means configured to reduce gradation of the brightness image data.

In the image processing apparatus described above, the scene determining means may be provided with a contour line image data generating means configured to generate contour line image data representing contour lines of brightness values based on the brightness image.

In the image processing apparatus described above, the contour line image data generating means may be provided with a vector differential calculating means configured to calculate gradient of the brightness image data.

In the image processing apparatus described above, the scene determining means may be provided with a contour line density calculating means configured to calculate a density of the contour lines, and the scene determining means may determine the photographic scene based on the density of the contour lines.

In the image processing apparatus described above, the scene determining means may be provided with a brightness gradient calculating means configured to calculate brightness gradient within the image, and the scene determining means may determine the photographic scene based on the brightness gradient.

In the image processing apparatus described above, the scene determining means may be provided with a circularity calculating means configured to calculate a circularity of a low brightness area of the image, and the scene determining means may determine the photographic scene based on the circularity.

In the image processing apparatus described above, the scene determining means may have a centroid calculating means configured to calculate a center of gravity of a low brightness area of the image, and the scene determining means may determine the photographic scene based on the centroid.

According to an embodiment of the present invention, a positional displacement between marks applied to an endoscopic image to indicate lesion parts and the endoscopic image itself can be prevented.

Brief Description of the Drawings

Fig. 1 is a block diagram schematically showing a configuration of an electronic endoscope apparatus according to an embodiment of the invention.
Fig. 2 is a block diagram schematically showing circuits regarding image processing of the electronic endoscope apparatus according to the embodiment of the invention.
Fig. 3 is a drawing schematically showing a configuration of storage areas of an image memory.
Fig. 4 is a flowchart illustrating a procedure of a process executed by an image processing circuit.
Fig. 5 shows an example of a gain curve used in a TE process.
Fig. 6 is a flowchart illustrating a procedure of an effective pixel judging process.
Fig. 7 is a flowchart illustrating a procedure of a lesion judging process.
Fig. 8 is a scatter diagram in which pixel values of biological tissues are plotted in an HS coordinate space.
Fig. 9 is a flowchart illustrating a procedure of a score calculating process.
Fig. 10 shows graphs showing relationships between a hue distance, a saturation distance and correlation values.
Fig. 11 shows a typical bright and dark distribution of an endoscopic image.
Fig. 12 is a flowchart illustrating a procedure of a scene determining process.
Fig. 13 is a flowchart illustrating a procedure of a moving image analyzing process.
Fig. 14 is a flowchart illustrating a procedure of an intensity gradient calculating process.
Fig. 15 illustrates the intensity gradient calculating process, and (a) is an example of an intensity image, (b) is an example of a graph of absolute values of gradient of intensity index, and (c) is an example of a contour image.
Fig. 16 is a flowchart illustrating a procedure of an inspection type determining process.
Fig. 17 shows an example of the marking image.
Fig. 18 is an example of a display screen generated by the display screen generating process.

**Embodiment for Carrying Out the Invention**

Hereinafter, referring to the drawings, embodiments of an image processing apparatus according to the present invention will be described. Incidentally, in the following description, an electronic endoscope system will be explained as one embodiment of the present invention.

[Entire Configuration of Electronic Endoscope Apparatus 1]

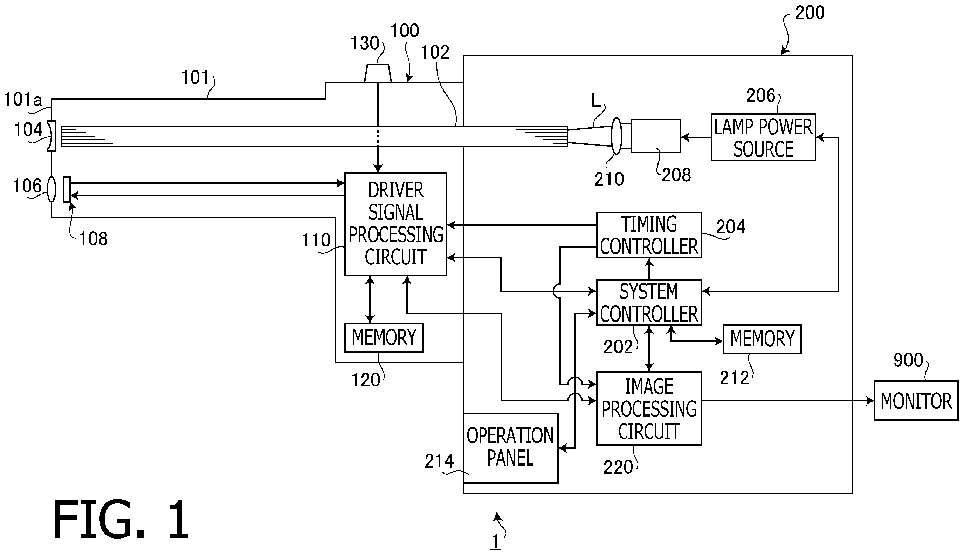

Fig. 1 is a block diagram showing a configuration of an electronic endoscope apparatus 1 according to the invention. As shown in Fig. 1, the electronic endoscope apparatus 1 is provided with an electronic scope 100, a processor 200 and a monitor 900.

The processor 200 is provided with a system controller 202 and a timing controller 204. The system controller 202 is configured to execute programs stored in a memory 212, and integrally control the electronic endoscope apparatus 1 entirely. The system controller 202 is connected to an operation panel 214. The system controller 202 changes operations of the electronic endoscope apparatus 1 and parameters for respective operations in accordance with instructions, which are input through the operation panel 214 by an operator. The timing controller 204 is configured to output synchronizing signals used to adjust operation timings of various parts to respective circuits of the electronic endoscope apparatus 1.

A lam 208 is actuated by a lamp power source igniter 206, and then, irradiates illuminating light L. The lamp 208 is, for example, a high-intensity lamp such as a xenon lam, a halogen lamp, a mercury lamp and a metal halide lamp, or an LED (light emitting diode). The illuminating light L is light having a spectrum ranging mainly from a visible light region to an invisible infrared region (or, white light including at least visible light region).

The illuminating light L irradiated by the lamp 208 is converged on an incident surface of an LCB (light carrying bundle) 102 by a converging lens 210, and enters into the LCB 102.

The illuminating light L entered the LCB 102 propagates inside the LCB 102, emitted from a light emitting surface of the LCB 102 which is arranged at a distal end of the electronic scope 100, and is incident on an object through a distribution lens 104. Return light from the object, which is illuminated by the illuminating light L, is converged, by an objective lens 106, to focus an optical image on a light receiving surface of a solid state imaging element 108.

The solid state imaging element 108 is a single CCD (charge coupled device) image sensor in accordance with a complementary color checkered color difference line sequential system). The solid state imaging element 108 picks up an optical image focused on the light receiving surface, and outputs an analog photographing signal. Specifically, the solid state imaging element 108 accumulates the optical image focused on respective pixels of the light receiving surface as electric charges corresponding to light amounts, generates yellow (Ye), cyan (Cy), green (G) and magenta (Mg) color signals, and sequentially outputs scan lines obtained by adding and mixing generated color signals of each two pixels arranged next to each other in a vertical direction. Incidentally, the solid state imaging element 108 needs not be limited to a CCD image sensor, but can be replaced with CMOS (complementary metal oxide semiconductor) image sensor, or any other type of imaging device. Further, the solid state imaging element 108 may be one mounting a primary color system filter (e.g., a Bayer array filter).

Inside a connection part of the electronic scope 100, a driver signal processing circuit 110 is provided. The analog photographing signal including the scan lines described above is input to the driver signal processing circuit 110 from the solid state imaging element 108 at a field period. Incidentally, in the following description, a term "field" could be replaced with a term "frame." In the embodiment, the field period and a frame period are 1/60 second and 1/30 second, respectively. The driver signal processing circuit 110 applies a predetermined processing to the analog photographing signal transmitted from the solid state imaging element 108, and outputs the same to an image processing circuit 220 of the processor 200.

The driver signal processing circuit 110 is also configured to access a memory 120 and retrieves intrinsic information which is intrinsic to the electronic scope 100. The intrinsic information of the electronic scope 100 recorded in the memory 120 includes, for example, the number of pixels, a sensitivity, an operable field rate, a model number of the solid state imaging element 108. The driver signal processing circuit 100 transmits the intrinsic information retrieved from the memory 120 to the system controller 202.

The system controller 202 executes various operations based on the intrinsic information of the electronic scope 100 to generates control signals. The system controller 202 controls operations and timings of circuits in the processor 200, with use of the generated control signals, so that processes suitable to the electronic scope connected to the processor 200 will be executed.

The timing controller 204 generates a synchronizing signal in accordance with a timing control by the system controller 202. The driver signal processing circuit 110 controls and drives the solid state imaging element 108, in accordance with the synchronizing signal supplied from the timing controller 204, at a timing synchronously with the field rate of a video signal generated by the processor 200.

The image processing circuit 220 generates image data based on the photographing signal output by the electronic scope 100, under control of the system controller 202. The image processing circuit 220 generates a screen data for monitor display using the generated image data, converts the screen data to a video signal having a predetermined video format, and outputs the same. The video signal is input to the monitor 900, and a color image of the object is displayed on a display screen of the monitor 900.

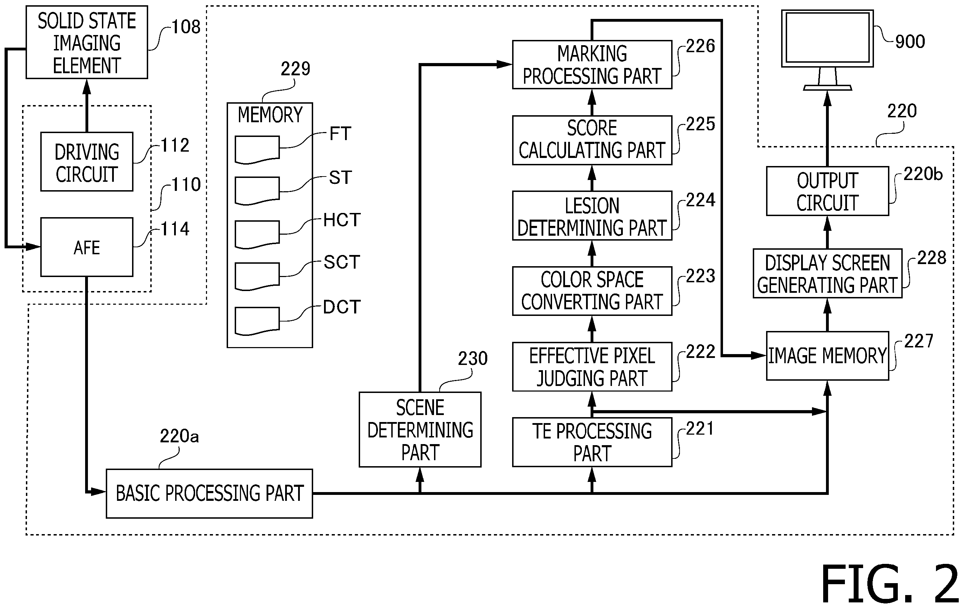

Fig. 2 is a block diagram schematically showing a configuration of a circuit regarding image processing executed by the electronic endoscope apparatus 1.

The driver signal processing circuit 110 is provided with a driving circuit 112 and an AFE (analog front end) 114. The driving circuit 112 generates a driving signal of the solid state imaging element 108 in accordance with the synchronizing signal. The AFE 114 applies noise reduction, signal amplification, gain compensation and A/D (analog to digital) conversion with respect to the analog photographing signal, and outputs a digital image signal, and outputs a digital photographing signal. Incidentally, all or a part of processing executed by the AFE 114 according to the embodiment may be executed by the solid state imaging element 108 or the image processing circuit 220.

The image processing circuit 220 is provided with a basic processing part 220a, an output circuit 220b, a TE (tone enhancement) processing part 221, an effective pixel judging part 222, a color space converting part 223, a lesion determining part 224, a score calculating part 225, a marking processing part 226, an image memory 227, a display screen generating part 228, a memory 229 and a reliability evaluating part 230. Processing executed by each part of the image processing circuit 220 will be described later.

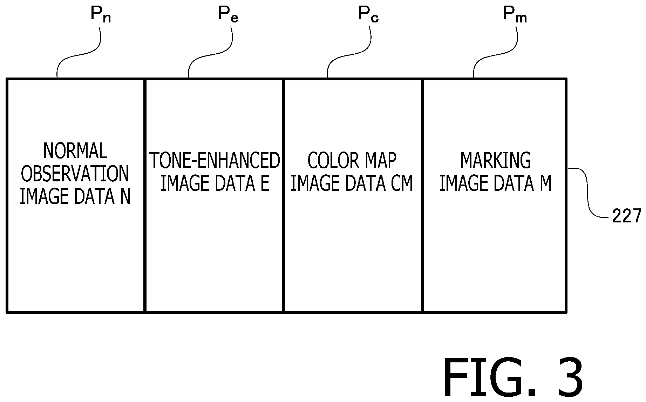

Fig. 3 schematically shows a configuration of storage areas allocated in the image memory 227. In the image memory 227 according to the embodiment, four storage areas Pn, Pe, Pm and Pc are allocated. The storage area Pn is an area which stores normal observation image data N (i.e., image data representing a normal observation image NP) which is generated by the basic processing part 220a. Incidentally, in the storage area Pn, two pieces or more of normal observation image data N subsequently generated can be stored. Further, writing/retrieving of data in/from the storage area Pn is performed in accordance with a first-in first-out (FIFO) method. The storage area Pe is an area which stores tone-enhanced image data E (i.e., image data representing a tone-enhanced image EP) generated by a TE processing part 221. The storage area Pc is an area which stores color map image data CM (i.e., an image data representing a color map image CMP) generated by the marking processing part 226. The storage area Pm is an area which stores marking image data M (i.e., image data representing a marking image MP) generated by the marking processing part 226.

As shown in Fig. 2, a flag table FT, a score table ST, a hue correlation value table HCT, a saturation correlation value table SCT, and a display color table DCT are stored in the memory 229. The flag table FT, and the score table ST are numeral value table having flags F (x, y) and scores Sc (s, y) representing analysis results regarding pixels (x, y) of the normal observation image data N, respectively. Specifically, the flags F (x, y) are parameters indicating presence/absence of lesions of tissues photographed on the corresponding pixels (x, y), the scores Sc (x, y) are parameters representing the severity degree of the lesions. The display color table DCT is a numerical value table defining a relationship between the scores Sc (x, y) and display colors (i.e., color codes) of the color map image CMP, which will be described later. The hue correlation value table HCT and the saturation correlation value table SCT will be described later.

[Basic Processing S1]

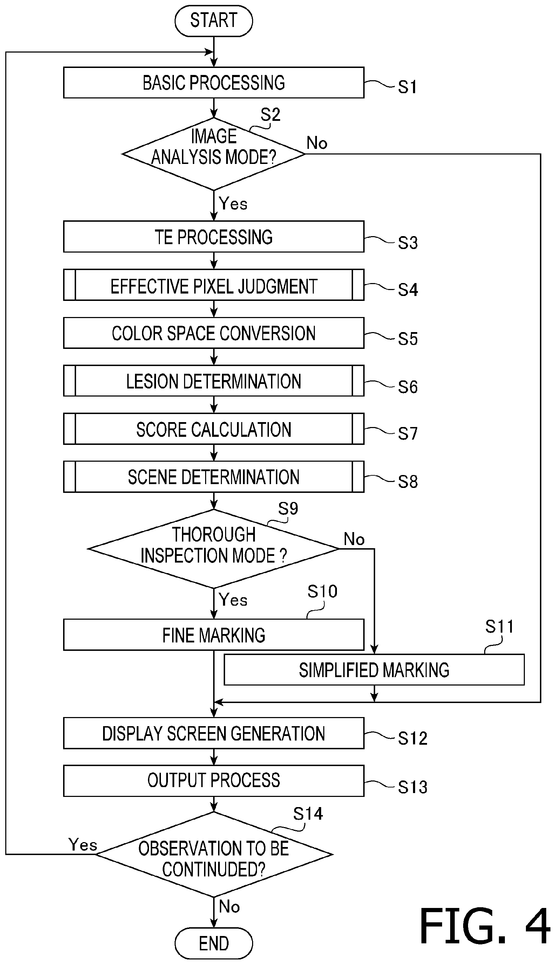

Next, processes executed by the image processing circuit 220 will be described. Fig. 4 is a flowchart illustrating procedures of the process executed by the image processing circuit 220. With respect to a digital signal output by the AFE 114, general signal processing (i.e., a basic processing S1) is applied by the basic processing part 220a, and normal observation image data N is generated.

The basic processing S1 includes a process of converting the digital photographing signal output by the AFE 114 to a intensity signal Y, and color difference signals Cb and Cr, a primary color separation process of separating primary colors R, G and B from the intensity signal Y, and color difference signals Cb and Cr, a clamp process of removing offset components, a defective pixel correction process of correcting a pixel value of a defective pixel with use of pixel values of surrounding pixels, a de-mosaic process (i.e., an interpolation process) of converting photographing data (i.e., RAW data) consisting of monochromatic pixel values to image data having full-color pixel values, a linear matrix process of correcting a spectral characteristic of the imaging element with use of a color matrix, a white balance process of compensating for spectral property of the illuminating light, and a contour correction process of compensating for deterioration of a spatial frequency characteristic.

Incidentally, all or part of the processes executed by the basic processing part 220a in the embodiment may be executed by the driver signal processing circuit 110 or the solid state imaging element 108.

The normal observation image data N generated by the basic processing part 220a is transmitted to the TE processing part 221 and the scene judging part 230, and further stored in the storage area Pn of the image memory 227.

[Operation Mode Judging Process S2]

Next, whether an operation mode is set to an image analysis mode (S2) is judged. The image analysis mode according to the embodiment of the invention is an operation mode in which color information is analyzed with respect each pixel of the image data, it is judged whether each pixel is a pixel photographing a lesion part (hereinafter, referred to as a lesion pixel) based on the result of analysis of the color information and a predetermined judging criteria, and the lesion pixels are displayed in a discriminated manner. Kinds of lesions to be judged can be selected depending on inspection contents. In an example described below, pixels of color range which is intrinsic to observation images of inflammation (e.g., reddening inflammation including selling or easy bleeding) of inflammatory bowel disease (IBD) are displayed in a discriminated manner.

It is noted that the electronic endoscope apparatus 1 according to the embodiment is configured to operate in either of two operation modes: an image analysis mode; and a normal observation mode. The operation mode is switched by a user operation to an operation part 130 of the electronic scope 100 or the operation panel 214 of the processor 200. When the operation mode is set to the normal observation mode (S2: NO), process proceeds to S12.

[TE (tone enhancement) Process S3]

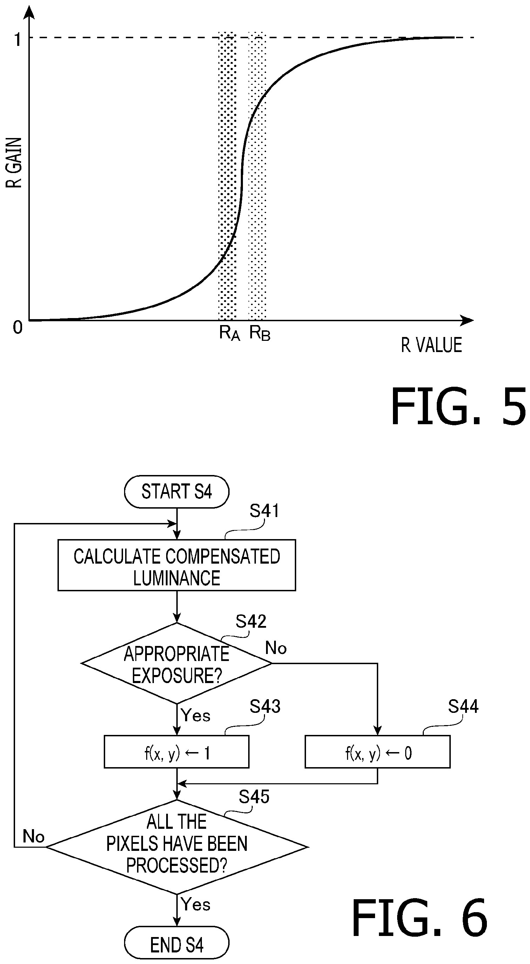

When the image analysis mode is selected (S2: YES), the TE process S3, which is to be executed by the TE processing part 221, is executed subsequently. The TE process S3 is a process of increasing an effective resolution by performing gain adjustment to give a non-linear gain to each of primary color signals R, G and B of the normal observation image data N, thereby substantially expanding a dynamic range in the vicinity of a characteristic color range (in particular, a boundary portion thereof) of the lesion subject to judgment. Specifically, in the TE process S3, a process of applying the non-linear gain as shown in Fig. 5 to each of primary color signals R, G and B to obtain primary color signals R', G' and B' (i.e., tone-enhanced image data E) is executed. For example, a gain curve shown in Fig. 5 is shaped such that an inclination of the curve is steep from a boundary range $R_A$, which is a characteristic color range of an ulcer, to a boundary region $R_B$, which is a characteristic color range of inflammation. By applying the gain in accordance with such a gain curve, a substantial dynamic range of the primary color signal R' (i.e., a signal obtained by applying the TE process S3 to the primary color signal R) from the boundary range $R_A$ to the boundary range $R_B$ can be expanded, thereby further precise threshold value judgment being enabled.

Incidentally, by the TE process S3, the hue changes such that the inflammatory part becomes reddish, the ulcer part becomes whitish and the normal part becomes greenish. Therefore, when the tone-enhanced image data E, which is generated in the TE process S3, is displayed on the monitor 900, lesion part (e.g., the inflammatory part or the ulcer part) can easily be visually recognized in comparison with a case where the normal observation image data N before the TE process S3 is applied is displayed. It is noted that the TE process S3 above is an example of a color enhancement process applicable to the present invention. Instead of the TE process S3, another type of color enhancement process capable of enhancing color quality, specifically, the hue or contrast of saturation (or chromaticity), may be employed.

[Effective Pixel Judging Process S4]

After the TE process S3 has completed, the effective pixel judging part 222 applies the effective pixel judging process S4 to the tone-enhanced image data E. It is noted that, the TE process S3 is omitted and the effective pixel judging process S4 may be applied to the normal observation image data N.

Fig. 6 is a flowchart illustrating a procedure of the effective pixel judging process S4. The effective pixel judging process S4 is a process of judging whether pixel values are suitable for image analysis, and is sequentially executed to all the pixels (x, y) constituting the image data. In the effective pixel judging process S4, firstly, for each pixel (x, y), based on the primary color signals R'(x, y), G'(x, y) and B'(x, y) of the tone-enhanced image data E, corrected intensity int(x, y) is calculated with use of formula 1 below.

[Formula 1]

$$\mathrm{int}(x, y) = 0.3 * R'(x, y) + 0.59 * G'(x, y) + 0.11 * B'(x, y)$$

Incidentally, values of the corrected intensity int(x, y) as calculated are used in a following appropriate exposure judging process S42. Further, as known from formula 1, the corrected intensity int(x, y) is not a simple average of the primary color signals R'(x, y), G'(x, y) and B'(x, y), but is obtained as a weighted average based on relative spectral sensitivity characteristic of human beings (e.g., the operator).

Next, for each pixel (x, y), the appropriate exposure judging process S42 is executed, in which whether the exposure level is appropriate to image analysis is judged based on the corrected intensity int(x, y) of the tone-enhanced image data E calculated in process S41 and the primary color signals R'(x, y), G'(x, y) and B'(x, y). In the appropriate exposure judging process S42, the exposure is determined to be the appropriate exposure (S42: YES) when at least one of (or both of) the following two conditions (i.e., formulae 2 and 3) is satisfied. Incidentally, formula 2 defines an upper limit value of the corrected intensity int(x, y) (the entire light amount), while formula 3 defines a lower limit value of each of the primary color signals R'(x, y), G'(x, y) and B'(x, y).

[Formula 2]

$$\mathrm{int}(x, y) < 235$$

[Formula 3]

$$Max\{R'(x, y), G'(x, y), B'(x, y)\} > 20$$

If, for the pixel (x, y), it is determined that formula 2 or formula 3 (or both formulae 2 and 3) is satisfied and the exposure is appropriate (S42: YES), the effective pixel judging part 222 rewrites the value of a flag F(x, y), which corresponds to the pixel (x, y), of the flag table FT stored in the memory 229 with value "1" (S43).

It is noted that the flag F (x, y) has a flag value of one of 0 - 2. Each flag value is defined below.

    0: invalid pixel data
    1: normal or unjudged (pixel data is valid)
    2: lesion (inflammation)

In the appropriate exposure judging process S42, if none of the formulae 2 and 3 is satisfied (or one of the formulae 2 and 3 is not satisfied), and the exposure is determined to be inappropriate (S42: NO), the effective pixel judging part 222 rewrites the value of the flag F(x, y) with "0" (S44).

In process S45, it is judged whether the process has been completed for all the pixels (x, y). Unless all the pixels (x, y) have been processed, the above processes S41 - S45 are repeated.

[Color Space Converting Process S5]

When the effective pixel judging process S4 has completed, the color space converting part 223 applies a color space converting process S5 to the tone-enhanced image data E. The color space converting process S5 is a process of converting pixel values of an RGB space defined by RGB three primary colors to pixel values of HIS (Hue-Saturation-Intensity) space defined by three elements of hue, saturation and intensity. Specifically, in the color space converting process S5, the primary color signals R'(x, y), G'(x, y) and B'(x, y) of each pixel (x, y) of the TE image data E is converted to hue H(x, y), saturation S(x, y) and intensity I(s, y).

Further, data of under or over exposure pixels (x, y) has low accuracy and lowers reliability degree of the analysis results. Therefore, the color space converting process S5 is applied only to the pixels (x, y) of which the value of the flag F(x, y) is set to be one (1) (i.e., the pixels (x, y) judged to be appropriately exposed in the effective pixel judging process S4).

Decision image data J{H(x, y), S(x, y), I(x, y)} having hue H(x, y), saturation S(x, y) and intensity I(x, y) of each pixel (x, y), which are generated by the color space converting part 223, is transmitted to the lesion determining part 224.

[Lesion Determining Process S6]

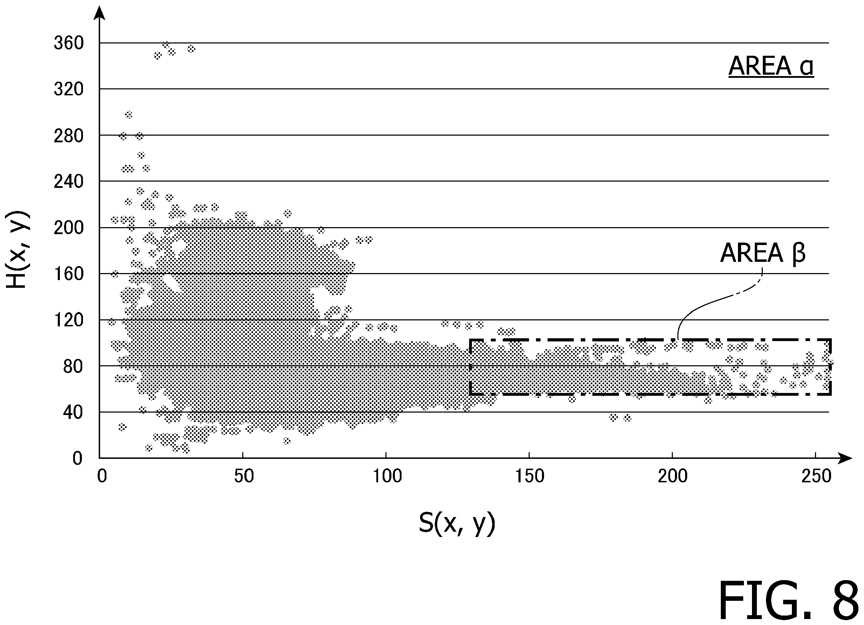

After completion of the color space conversion process S5, the lesion determining part 224 executes a lesion judging process S6 using the decision image data J. the lesion determining process S6 is a process applied to each pixel (x, y) of the endoscope image, in which process a condition of the biological tissue photographed by the pixel is determined (i.e., it is judged whether the biological tissue is in the inflammatory condition) depending on whether the decision image data J is plotted on which of areas α or β (see Fig. 8; described later) in an HS space (i.e., Hue-Saturation space). It is noted that the HS space is, similar to the a chromaticity space, a space representing quality of colors (i.e., components excluding brightness/intensity). For example, when the image analysis is performed on another color space such as a CIE 1976 L\*a\*b\* color space, lesion determination by the lesion determining part 224 is executed on the chromaticity space (e.g., an a\*b\*space).

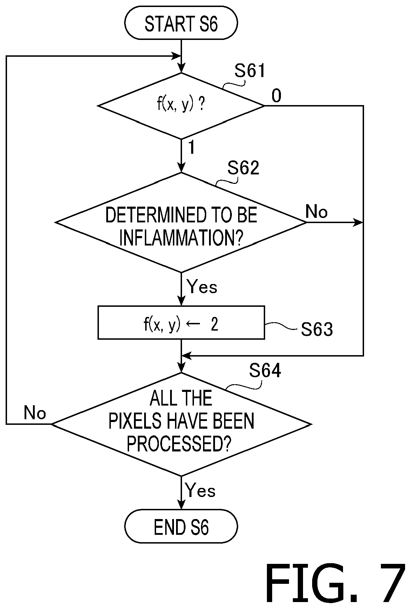

Fig. 7 is a flowchart illustrating a procedure of the lesion determining process S6. The lesion determining process S6 is executed for all the pixels (x, y) constituting the image data, sequentially. In the lesion determining process S6, firstly, it is determined whether data of each pixel (x, y) is valid, referring to the flag table FT (S61). When the value of the flag $F(x, y)$ is "1" (i.e., the pixel data is valid), an inflammation determining process S62 is executed. When the value of the flag $F(x, y)$ is "0" (i.e., the pixel data is invalid), control proceeds to process S64 without executing the inflammation determining process S62.

The inflammation determining process S62 will be described. Fig. 8 is a scatter diagram which shows that decision image data J obtained from the endoscope image data of a plurality of inflammatory bowel disease patients is plotted in an HS coordinate space.

The scatter diagram shown in Fig. 8 is classified into area β which is located on a lower right portion and encircled by broken lines, and area α which is an area other than area β. According to the research of the inventors of the present invention, it has become clear that most of the portions determined as inflammatory portions by doctors who are skilled in endoscopic diagnosis of the inflammatory bowel disease are plotted in area β, while most of the portions determined as non-inflammatory portions by the doctors skilled in endoscopic diagnosis of the inflammatory bowel disease are plotted in area α. From the above, the condition of the biological tissue (i.e., presence/absence of the inflammation) can be judged with sufficient accuracy based on the two pieces of information of the hue (shade of color) and saturation (vividness of color) of the endoscopic observation image photographing biological tissues.

In the inflammation determining process S62, it is determined whether decision image data $J\{H(x, y), S(x, y)\}$ of each pixel (x, y) is to be plotted in area β shown in Fig. 8. Specifically, the decision image data $J\{H(x, y), S(x, y)\}$ is plotted in area β when both formulae 4 and 5 below are satisfied. When the decision image data $J\{H(x, y), S(x, y)\}$ does not satisfy at least one of formulae 4 and 5, the decision image data $J\{H(x, y), S(x, y)\}$ is plotted in area α (i.e., it is determined that the pixels are not those of the inflammatory portions). Incidentally, $\delta_{S1}$, $\delta_{H1}$ and $\delta_{H2}$ are compensation values which can be set by the operator, and by the settings of these compensation values, strictness of decision (i.e., sensitivity) can be appropriately adjusted.

[Formula 4]

$$130 + \delta_{S1} \leq S(x, y)$$

[Formula 5]

$$60 + \delta_{H1} \leq H(x, y) \leq 100 + \delta_{H2}$$

When the decision image data $J\{H(x, y), S(x, y)\}$ of a pixel (x, y) is plotted in area β (S62: YES), the value of the flag $F(x, y)$ corresponding to the pixel (x, y) is rewritten with "2" (i.e., inflammation) (S63), and control proceeds to process S64. When the decision image data $J\{H(x, y), S(x, y)\}$ of a pixel (x, y) is not plotted in area β (S62: NO), the flag $F(x, y)$ is not rewritten, and control proceeds to process S64.

In process S64, it is judged whether all the pixels (x, y) have been processed. Until all the pixels (x, y) are processed, above processes S61 - S64 are repeated.

[Score Calculating Process S7]

After the lesion determining process S6 has completed, a score calculating process S7 is executed. The score calculating process S7 is a process of calculating a score $Sc(x, y)$ representing an evaluation value of severity degree of the lesion part based on the pixel values of the decision image data J. The score calculating process S7 is executed sequentially for all the pixels (x, y). Incidentally, an algorithm of the score calculation explained below is only an example, and the present invention can be applied to displayed screens of scores calculated in various algorithms, respectively.

(Principle of Score Calculation)

Here, a principle of score calculation according to the embodiment will be described briefly. It is known that the more a symptom of an inflammatory part progresses, the closer the color of the inflammatory part becomes the color of blood as superficial normal mucous membranes will be fallen out. Therefore, degree of correlation between the color of the inflammatory part and the color of the blood (i.e., correlation value CV, which will be described later) serves as a good index representing the severity degree of the inflammatory part. According to the present embodiment, the correlation value CV(x, y) representing the relative correlation between the decision image data J{H(x, y), S(x, y)} of each pixel (x, y) and a color of the blood (i.e., hue and saturation) is calculated, which is used as the score Sc(x, y) representing the severity of the inflammatory part.

(Lesion Part Judgment S71)

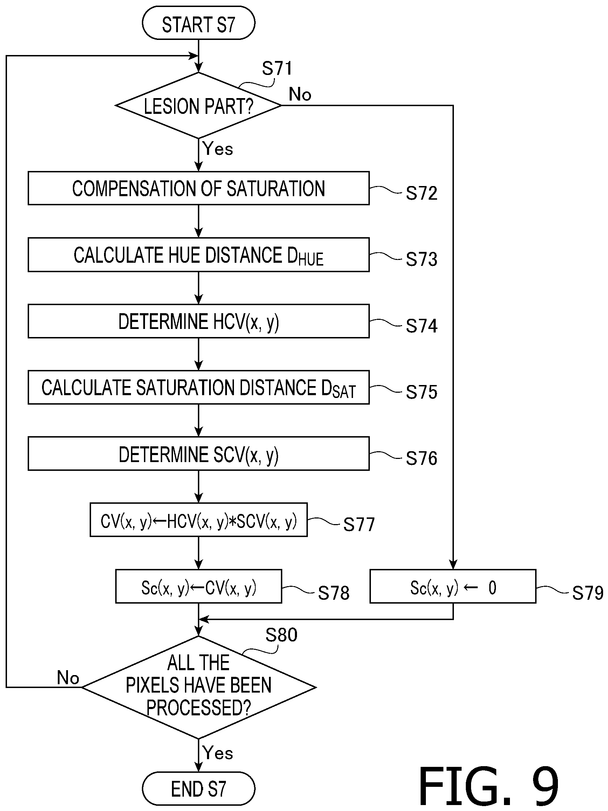

Fig. 9 is a flowchart illustrating a procedure of the score calculation process S7. In the score calculation process S7, the flag table FT is firstly retrieved, and it is judged whether the value of the flag F(x, y) corresponding to the pixel (x, y) is "2" (i.e., inflammation) (S71).
When the value of the flag F(x, y) is "2" (inflammation), namely, when the pixel (x, y) is the lesion pixel (S71: YES), process proceeds to S72. When the pixel (x, y) is not the lesion pixel (S71: NO), process proceeds to S79.

(Compensation of Saturation: S72)

It is known that saturation of blood or biological tissue including blood depends on its intensity. Specifically, saturation thereof is lower as the intensity is higher. In S72, variation of saturation S(x, y) due to intensity I(x, y) of the decision image data J(x, y) is compensated using formula 6 which is developed by the present inventors. By applying this compensation, it is possible to make precision of score calculation higher.

[Formula 6]

$$\begin{bmatrix} I_{corr.}(x, y) \\ S_{corr.}(x, y) \end{bmatrix} = \begin{bmatrix} \cos\theta & -\sin\theta \\ \sin\theta & \cos\theta \end{bmatrix} \begin{bmatrix} I(x, y) \\ S(x, y) \end{bmatrix} + \begin{bmatrix} I_{ref} \\ S_{ref} \end{bmatrix}$$

where,

Icorr.(x, y): luminance of the decision image data J after compensation;
Scorr.(x, y): saturation of the decision image data J after compensation;
Iref.: luminance of blood sample data serving as a reference value; and
θ: an angle providing with a correlation index (cos θ) between the saturation and the luminance of the blood sample.

It is noted that the correlation index (measured value) is -0.86, and accordingly, θ=149.32(degree) is used.

(Calculation of Hue Distance D$_{HUE}$: S73)

Next, using formula 7, a hue distance D$_{HUE}$(x, y) is calculated (S73). The hue distance D$_{HUE}$ is a relative value of the hue of the decision image data J(x, y) using the hue H$_{ref}$ of the blood sample data as reference.

[Formula 7]

$$D_{HUE}(x, y) = H(x, y) - H_{ref}$$

(Determination of hue correlation value HVC: S74)

Next, a hue correlation value HVC(x, y) is determined (S74) based on the hue distance D$_{HUE}$(x, y). The hue correlation value HCV(x, y) is a parameter having strong correlation with severity degree of an inflammation part. Fig.

10(a) is a graph showing a relationship between the hue distance $D_{HUE}$ and the hue correlation value HCV. The hue distance $D_{HUE}$ exhibits a strong correlation with the severity degree of the inflammation part within a range of $\pm 30°$ (hereinafter, referred to as a "hue approximation range $R_{11}$"), while exhibits little correlation in other ranges. Therefore, the hue correlation value HCV(x, y) of the present embodiment is set to a minimum value of 0.0 in a non-hue approximation range $R_{12}$, and set to linearly increase as the hue distance $D_{HUE}(x, y)$ approaches 0° within the hue approximation ranges $R_{11}$. Further, the hue correlation value HCV(x, y) is normalized such that the minimum and maximum values of the hue correlation values HCV(x, y) are 0.0 and 1.0, respectively.

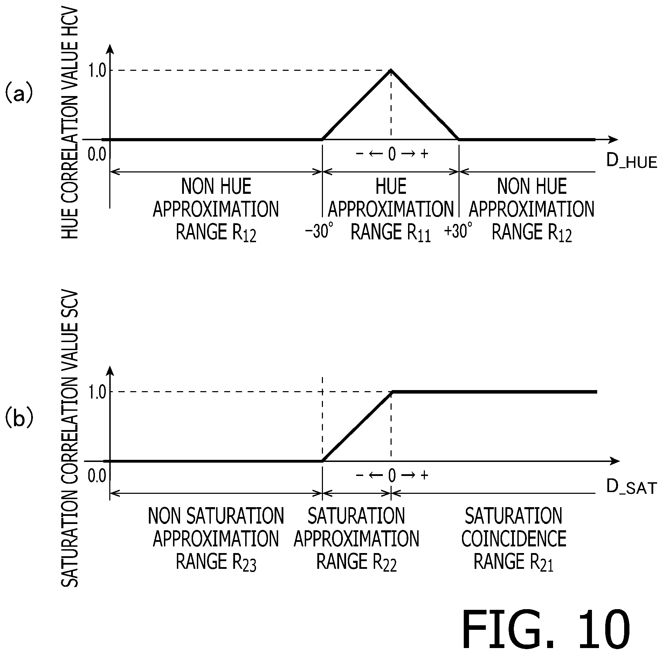

The relationship between the hue distance $D_{HUE}$ and the hue correlation value HCV shown in Fig. 10(a) is stored in the memory 229 in form of a hue correlation value table HCT. By referring to the hue correlation value table HCT, a hue correlation value HCV(x, y) corresponding to a hue distance $D_{HUE}(x, y)$ can be obtained.

(Calculation of Saturation Distance: S75)

Next, a saturation distance $D_{SAT}(x, y)$ is calculated using formula 8. The saturation distance $D_{SAT}(x, y)$ is a relative value of saturation of the decision image data J(x, y) using saturation $S_{ref}$ of the blood sample data as reference.

[Formula 8]

$$D_{SAT}(x, y) = S_{corr.}(x, y) - S_{ref}$$

[Determination of Saturation Correlation Value SCV: S76]

Next, a saturation correlation value SCV(x, y) is determined based on the saturation distance $D_{SAT}(x, y)$ (S76). The saturation correlation value SCV(x, y) is also a parameter having strong correlation with the severity degree of the inflammation part. Fig. 10(b) is a graph showing a relationship between the saturation distance $D_{SAT}(x, y)$ and the saturation correlation value SCV. The saturation distance $D_{SAT}(x, y)$ has strong correlation with the severity degree of the inflammation part in a negative range in which the saturation distance $D_{SAT}$ has a value equal to or greater than a predetermined value (hereinafter, referred to as a saturation approximation range $R_{22}$), while the saturation distance $D_{SAT}$ has little correlation in a negative range and the saturation distance $D_{SAT}$ has a value equal to or less than the predetermined value. Further, in a range in which the saturation distance $D_{SAT}$ is zero or greater, that is, in a range where the saturation of the lesion pixel is equal to or greater than the saturation Sref of the blood sample data (hereinafter, referred to as saturation coincidence range $R_{21}$), it is considered that the severity degree is quite high. Therefore, the saturation correlation value SCV(x, y) according to the present embodiment is configured such that the saturation correlation value SDV(x, y) is set to have the maximum value of 1.0 within the saturation coincidence range $R_{21}$, set to have the minimum value of 0.0 within the non-saturation approximation range $R_{23}$, and set to linearly increase within the saturation approximation range $R_{22}$. It is noted that the saturation correlation value SCV(x, y) is also a normalized value which has the minimum value of 0.0 and the maximum value of 1.0.

The relationship between the saturation distance $D_{SAT}$ and the saturation correlation value shown in Fig. 10(b) is stored in the memory 229 in form of a saturation correlation value table SCT. By referring to the saturation correlation table SCT, a saturation correlation value SCV(x, y) corresponding to a saturation distance $D_{SAT}(x, y)$ can be obtained.

(Calculation of Correlation Value: S77)

Next, by multiplying the hue correlation value HCV(x, y) with the saturation correlation value SCV(x, y), a correlation value CV(x, y) between the color of a lesion pixel (x, y) and the color of blood. It is noted that the correlation value CV(x, y) is a normalized value of which the minimum value is 0.0 and the maximum value is 1.0. Further, the correlation value CV(x, y) is divided into eleven steps with a pitch of 0.1 point.

(Update of Score Sc: S78)

Since the correlation value CV(x, y) serves as an appropriate index of severity degree of the inflammation, the value of the score Sc(x, y) in the score table ST is rewritten with the correlation value CV(x, y) (S78).

(Updating of Score Sc: S79)

When a pixel (x, y) is not the lesion pixel (S71: NO), the above-described calculation of the correlation value CV(x, y) is not executed, and the value of the score Sc(x, y) in the score table ST is rewritten with "0" (S79). According to this configuration, scores Sc(x, y) can be given to all the pixels (x, y) with a smaller amount of calculations.

In process S80, it is judged whether the processing has been completed for all the pixels (x, y). Until processing has been completed for all the pixels (x, y), above-described processes S71 - S80 are repeated.

[Scene Determination: S8]

After completion of the score calculation S7 (or, in parallel with a series of processes from the TE processing S3 to the score calculation process S7), a scene determining process S8 is executed by the scene determining part 230.

The scene determining process S8 according to the present embodiment will be generally described below. Generally, endoscopic inspections are executed by roughly breaking down into two steps (i.e., two kinds of inspections). A first step is a "screening inspection" to search for portions suspected to be lesion parts by observing an inspection object (e.g., inner walls of an esophagus, a stomach and a duodenal in case of an upper gastrointestinal endoscopy) throughout. A second step is a "thorough inspection" in which the suspected portions found in the screening inspection are thoroughly observed to judge lesion tissues/normal tissues, and when the found portions are lesion tissues, a kind and the severity degree thereof are judged.

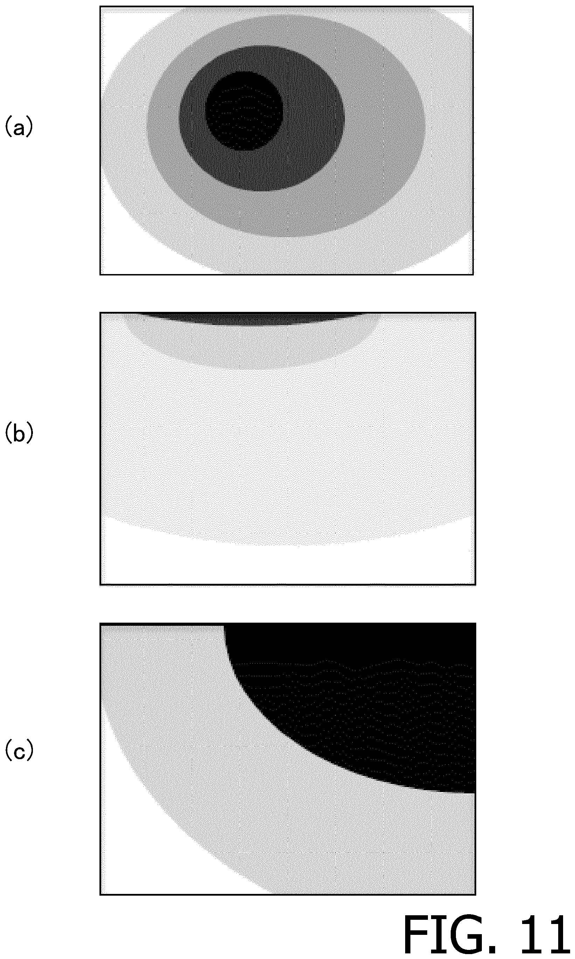

Fig. 11 shows a typical bright and dark distribution of an endoscopic image. Fig. 11(a) shows an example of a typical bright and dark distribution of an endoscopic image when the screening inspection is executed, and Figs. 11(b) and (C) show typical bright and dark distributions in the endoscopic image when the thorough inspection is being executed.

[TABLE 1]

|  | IMAGE MOVEMENT | LOCATION OF DARK PART (SHAPE) | INTENSITY GRADIENT |
|---|---|---|---|
| SCREENING INSPECTION | FAST | CENTRAL PART (CIRCULAR) | LARGE |
| THOROUGH INSPECTION | SLOW | PERIPHERAL PART (NON-CIRCULAR) | SMALL |

Incidentally, Table 1 shows features of the endoscopic images photographed when the screening inspection is executed (see Fig. 11(a)) and when the thorough inspection is executed (Fig. 11(b)) in a contrasted manner.

In an ordinary screening inspection, a tip 101a (see Fig. 1) of an insertion part 101 of the electronic scope 100 is once inserted into an innermost part of an inspection area in a gastrointestinal tract, and an observation is carried out through the entire length of the inspection area with gradually drawing the insertion part 101 from the gastrointestinal tract. It is noted that the observation in the screening inspection is carried out with aiming the tip 101a of the insertion part 101 slightly toward a wall side of the gastrointestinal tract and scanning a field of vision in a circumferential direction of the gastrointestinal tract. Since an inspection target of the screening inspection should cover a wide area, the inspection should be carried out efficiently. Therefore, a distance between the tip 101a of the electronic scope 100 and the object (e.g., a wall of the gastrointestinal tract) is maintained to be an intermediate - long distance. Specifically, the screening inspection is carried out with the insertion part 101 of the electronic scope 101 being substantially parallel with each other (or, the tip 101a being slightly aimed toward the wall of the gastrointestinal tract).

Therefore, in the endoscopic image photographed when the screening inspection is carried out, a dark inner wall of the gastrointestinal tract spaced from the tip 101a of the electronic scope 100 is shown at a central part of the image, and brightly illuminated inner wall of the gastrointestinal tract close to the tip 101a of the electronic scope 100 is shown at a peripheral part of the image as shown in Fig. 11(a). Further, the dark (i.e., low intensity) part in the endoscopic image is substantially circular.

Further, in the image in the screening inspection, from a brightly illuminated part close to the tip 101a to a dark and distant part to which almost no illumination light reaches are shown, change of darkness/brightness (i.e., intensity) in the image becomes large.

Further, since the screening inspection is carried out with moving the tip 101a of the electronic scope 100 as described above, movement of the endoscopic image photographed during the screening inspection is fast.

The thorough inspection is carried out by aiming the tip 101a of the electronic scope 100 close to a particular part of the inner wall of the gastrointestinal tract (i.e., a part which is suspected to be a lesion part in the screening inspection). Therefore, in the endoscopic image photographed when the thorough inspection is carried out, a brightly illuminated wall of the gastrointestinal tract close to the tip 101a of the electronic scope 100 is shown at a central part (or substantially entire part), while a dark inner wall of the gastrointestinal tract distant from the tip 101a is shown in a peripheral part of the image. Accordingly, the dark part in the endoscopic image has a non-circular shape.

It is noted that the thorough inspection is carried out such that movement of the tip 101a of the electronic scope 100 is as small as possible in order to observe minute shape and texture of the object. Therefore, movement of the endoscopic image photographed during the thorough inspection is slow and gentle.

Since dark part which is distant from the tip 101a of the electronic scope 100 is not substantially photographed in the image of the through inspection, change of brightness/darkness within the image is gentle.

The scene determining process S8 according to the present embodiment is a process of determining a status of inspection (i.e., whether the screening inspection is being executed or the thorough inspection is being executed) based on the features (in particular, the movement of the image) described in Table 1.

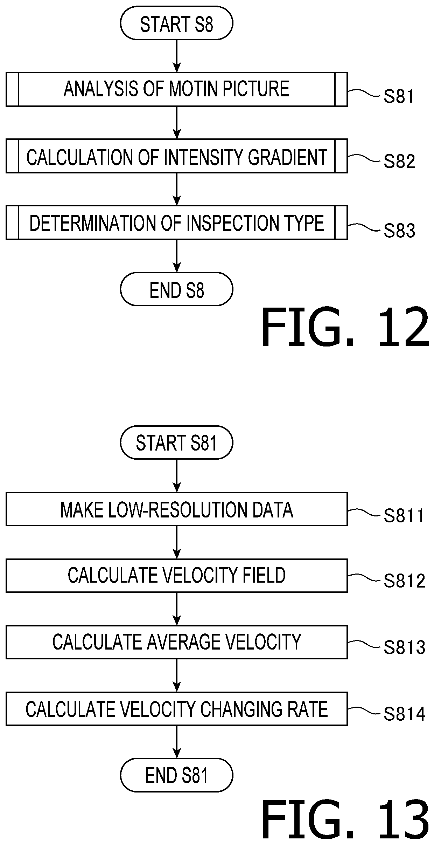

Fig. 12 shows a flowchart illustrating a procedure of the scene determining process. The scene determining process S8 includes a motion picture analyzing process S81 to analyze movement of the endoscopic image, an intensity gradient calculating process S8 to analyze a brightness/darkness distribution within the endoscopic image, and an inspection type determining process S83 to determine the type of inspection (i.e., the screening inspection/the thorough inspection) based on the analysis results of the motion picture analyzing process S81 and the intensity gradient calculating process S82.

(Analysis of Motion Picture: S81)

Fig. 13 is a flowchart illustrating a procedure of the motion picture analyzing process S81.

(Low-Resolution Data Making: S811)

In the motion picture analyzing process S81, firstly, a low-resolution data making process S811 to convert the normal observation image NP to a low-resolution normal observation image $NP_r$ by reducing the resolution (i.e., the number of pixels) of the normal observation image NP to $1/n^2$ thereof (n being an integer) is executed. This process is aimed to reduce calculation amounts required in the following steps, and according to the present embodiment, the resolution of the normal observation image NP is reduced to 1/16 thereof. Specifically, the normal observation image NP is divided into blocks each has n pixels X n pixels (e.g., four pixels by four pixels), and n2 pixels (e.g., 16 pixels) in each block is integrated into a new single pixel. In that instance, a representative value of the pixel values N(x, y) is calculated for each block (e.g., an average value, a median value or a most frequent value of the pixel values N(x, y) of each block), and the representative value is used as the pixel value of the low-resolution normal observation image $NP_r$.

(Calculation of Velocity Field: S812)

Next, based on the low-resolution normal observation image $NP_r$ of the latest frame and that of the previous frame, a velocity vector field {$V_x$(x, y), Vy(x, y)} (hereinafter, simply referred to as a velocity filed (Vx, Vy)) is calculated (S812). This velocity field is an optical flow calculated in accordance with, for example, a gradient method or Lucas-Kanade method.

(Calculation of Average Velocity: S813)

Next, with use of formula 9, an image velocity PV which is a root mean square if the velocity field (Vx, Vy). The image speed PV is a parameter indicative of quantity of an average velocity of an entire image.

[Formula 9]

$$ PV = \sqrt{\frac{1}{N_v}\Sigma\left[V_x^2(x,y) + V_y^2(x,y)\right]} $$

where, Nv: the number of elements of the velocity field (i.e., the number of pixels of the low-resolution normal observation image $NP_r$).

(Calculation of Changing Rate of Image Velocity: S814)

Next, an image velocity changing rate PV' (i.e., a changing amount of the image velocity PV per unit time) which is a time differential of the image velocity PV is calculated. Further, a smoothing process is applied to the image velocity changing rate PV'. Specifically, a representative value (e.g., the average value, a median value or a most frequent value) of the image velocity changing rates PV' for multiple low-resolution normal observation images $NP_r$ photographed within a latest predetermined time period (e.g., for one second) are calculated, and the representative value is used as the image speed changing rate PV'. It is noted that a value obtained simply by applying the time differential to the image velocity PV includes large amounts of noises of high-frequency components (e.g., movement of image due to oscillation and the like, which is not expected by the operator, of the electronic scope 100). Therefore, if the smoothing process is not applied, but the time differential value of the image velocity PV is used as is and the scene determining process S83 is executed, the determination result becomes unstable, which causes frequent changes of the display modes.

(Intensity Gradient Calculation: S82)

After the moving image analyzing process S81, an intensity gradient calculating process S82 to calculate an intensity gradient (i.e., the maximum value $LD_{max}$ of a density $LD_\varphi$ which will be described later) in the normal observation image NP is executed.

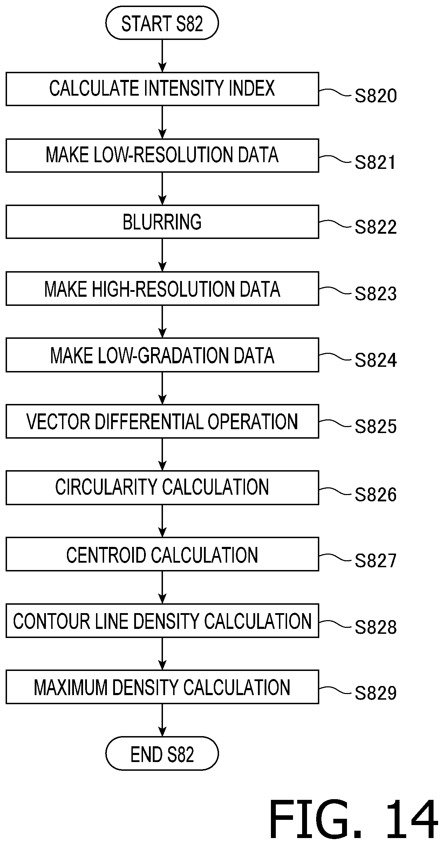

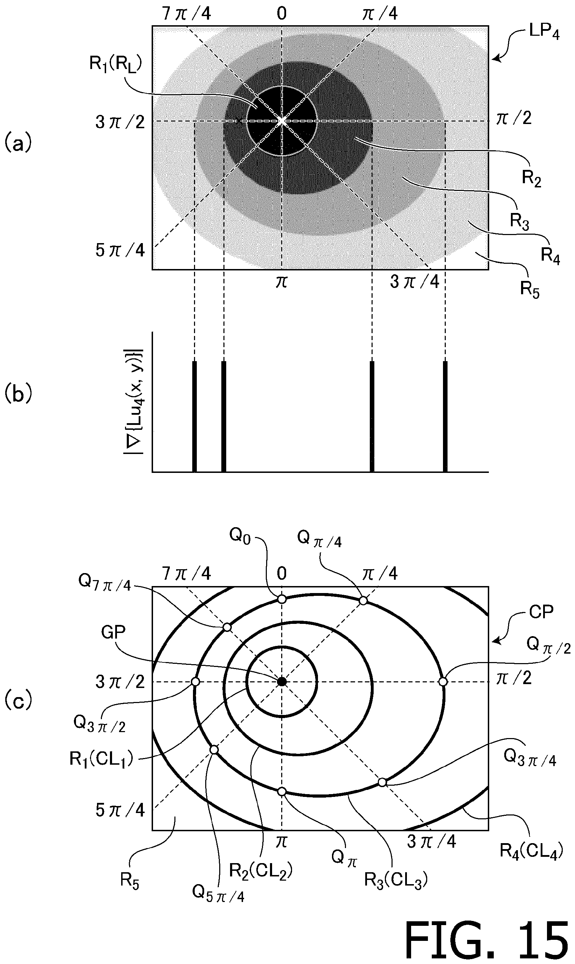

Fig. 14 is a flowchart illustrating a procedure of the intensity gradient calculating process S82. Fig. 15 shows the intensity gradient calculating process S82. Fig. 15(a) is an example of an intensity image $LP_4$[intensity index $Lu_4(x, y)$], Fig. 15(b) is an example of a graph showing absolute values of gradient of the intensity index $Lu_4(x, y)$, and Fig. 15(c) is an example of contour line image CP which will be described later.

(Intensity Index Calculation: S820)

In the intensity gradient calculating process S82, an intensity index $Lu0(x, y)$ of each pixel $(x, y)$ of the normal observation image NP is firstly calculated with use of formula 10, and intensity image data $Lu_0$ (i.e., image data representing the intensity image $LP_0$) which has the intensity indexes $Lu_0(x, y)$ as elements (pixel values) is generated (S820). It is noted that, according to the present embodiment, the intensity indexes $Lu_0(x, y)$ are calculated as a simple average of the values of primary color signals $R(x, y)$, $G(x, y)$ and $B(x, y)$ of respective pixels of the normal observation image data N. However, the intensity indexes $Lu_0(x, y)$ may be calculated by weighted average corresponding to a spectral sensitivity characteristic of the solid state imaging elements 108, or by weighted average corresponding to the relative spectral sensitivity characteristic as is treated in formula 1. Alternatively, the intensity indexes $Lu_0(x, y)$ may be calculated not as the average of the primary color signals, but a sum of the same.

[Formula 10]

$$Lu(x, y) = \{R(x, y) + G(x, y) + B(x, y)\}/3$$

(Making Low-Resolution Data: S821)

Next, a resolution decreasing process S821 to convert intensity image data $Lu_0$ to intensity image data $Lu_1$ (i.e., image data representing an intensity image LP1) having intensity indexes $Lu_1(x, y)$ as elements (i.e., pixel values) by decreasing the resolution (the number of pixels) of the intensity image data $Lu_0$ to $1/n^2$. The resolution decreasing process S821 reduces the amount of calculations required in respective steps in the following steps, and further, simplifies the intensity image $Lu_0$.

(Blurring: S822)

Next, a blurring process S822 is executed. In the blurring process S822, for each pixel, a representative value (e.g., an average value, a median value or a most frequent value) of the intensity indexes $Lu1(x, y)$ of the pixels included in a predetermined area (e.g., 3 pixels by 3 pixels) centered on the pixel, and the intensity image data $Lu_2$ (i.e., image data representing the intensity image $LP_2$) having the representative values (i.e., intensity indexes $Lu2(x, y)$) as its elements (i.e., pixel values) is generated. The blurring process S822 further simplifies the intensity image $LP_1$.

(Making High-Resolution Data: S823)

Next, a resolution increasing process S823 to increase the resolution (the number of pixels) of the intensity image data $Lu_2$ by multiplying $n^2$ (n being an integer) to generate intensity image data $Lu_3$ (i.e., image data representing the intensity image $LP_3$) of which resolution is returned to that of the original intensity image dataLu0 is executed. The resolution increasing process S823 is executed by dividing each pixel into n pixels by n pixels. By the resolution increasing process, the resolution (i.e., the number of pixels) increases, however, the image itself does not change.

(Making Low-Gradation Data: S824)

Next, intensity image data $Lu_4$ (i.e., image data representing an intensity image LP4 is generated as a gradation decreasing process S824 to decrease the gradation of the pixel values is executed with respect to the intensity image data $Lu_3$. In the gradation decreasing process S824, for example, the gradation is decreased from 256 steps to 8 steps or 16 steps.

By the gradation decreasing process S821, the blurring process S822, the resolution increasing process S823 and the resolution decreasing process S824, simplification of the intensity image $LP_0$ is executed effectively. Incidentally, by applying Fourier transformation to the intensity image data $Lu_0$ to eliminate high-frequency components, then by applying inverse Fourier transformation thereto instead of executing the above processes, the intensity image $LP_0$ can be similarly simplified. Further, by executing the blurring process S822 by multiple times, substantially the similar effects can be obtained.

(Vector Differential Operation: S825)

Next, a vector differential operating process S825 is applied with respect to the intensity image data $Lu_4$. Specifically, the gradient of the intensity indexes $Lu_4(x, y)$ is calculated.

As shown in Fig. 15(a), the intensity image $LP_4$ has a very simple stepwise structure configured with multiple areas of which intensities are different (five areas $R_1$, $R_2$, $R_3$, $R_4$ and $R_5$ in Fig. 5). When the gradient is calculated based on this intensity image LP4 (intensity indexes Lu4(x, y)), as shown in Fig. 15(b), only at boundaries of the areas, values having certain magnitudes are obtained (i.e., gradient at positions other than the boundaries is zero). Therefore, by the vector differential operating process S825, contour line image data CD representing the boundaries of the areas of respective gradients of the intensity image LP4, that is, contour lines CL of the intensities (i.e., a contour line image CP) can be obtained.

(Circularity Calculation: S826)

Next, using the contour line image data CD, a circularity $V_{round}$ of the darkest image area (i.e., a lowest gradation area $R_L$) in the intensity image $LP_4$ is calculated with use of formula 11 (S826).

[Formula 11]

$$V_{round} = \frac{4\pi N_{RL}}{L_{RL}^2}$$

where,

$N_{RL}$: the number of pixels (i.e., area) within the lowest gradation area $R_L$
$L_{RL}$: the length of the contour line (i.e., circumferential length) of the lowest gradation area $R_L$

(Centroid Calculation: S827)

Next, using the contour line image data CD, the centroid GP of the lowest gradation area $R_L$ is calculated with use of Formula 12 (S827).

[Formula 12]

$$GP = \left( \frac{x_{RL\min} + x_{RL\max}}{2}, \frac{y_{RL\min} + y_{RL\max}}{2} \right)$$

where,

$X_{RLmin}$: the minimum value of the X-coordinate in the lowest gradation area $R_L$
$X_{RLmax}$: the maximum value of the X-coordinate in the lowest gradation area $R_L$
$Y_{RLmin}$: the minimum value of the Y-coordinate in the lowest gradation area $R_L$
$Y_{RLmax}$: the maximum value of the Y-coordinate in the lowest gradation area $R_L$

(Density Calculation: S828)

Next, using the contour line image data CD, densities LD of the contour lines CL in eight directions (0, $\pi/4$, $\pi/2$, $3\pi/4$, $\pi$, $5\pi/4$, $3\pi/2$, $7\pi/4$) starting at the centroid GP are calculated (S828). The density LD of the contour lines CL is defined as the number of contour lines CL per a unit length in a radial direction with respect to the centroid GP.

In the intensity image $LP_4$ shown in Fig. 15(a) and in the contour line image CP shown in Fig. 15(c), from one having the lowest intensity, five areas $R_1$ (the minimum gradation area $R_1$), $R_2$, $R_3$, $R_4$ and $R_5$ are photographed. Among these areas, the whole circumferences of the contour lines $CL_1$, $CL_2$ and $CL_3$ of the areas $R_1$, $R_2$ and $R_3$ having loser intensities are photographed in the contour line image CP. According to the present embodiment, the densities LD of the contour lines LD are calculated using only the contour lines $CL_1$, $CL_2$ and $CL_3$ of which the whole circumferences (or, at least respective points in the eight directions in which the densities are calculated) are photographed in the contour line image CP.

In the calculation of the density LD of the contour lines CL, firstly, intersections $Q_0$, $Q_{\pi/4}$, $Q_{\pi/2}$, $Q_{3\pi/4}$, $Q_{\pi}$, $Q_{5\pi/4}$, $Q_{3\pi/2}$, $Q_{7\pi/4}$ of radial lines (broken lines) respectively extending eight directions from the centroid GP and the outermost contour line $CL_3$ are detected. Then, distances do, $d_{\pi/4}$, $d_{\pi/2}$, $d_{3\pi/4}$, $d_{\pi}$, $d_{5\pi/4}$, $d_{3\pi/2}$, $d_{7\pi/4}$ (not shown) between the centroid GP and the respective intersections $Q_0$, $Q_{\pi/4}$, $Q_{\pi/2}$, $Q_{3\pi/4}$, $Q_{\pi}$, $Q_{5\pi/4}$, $Q_{3\pi/2}$, $Q_{7\pi/4}$ are calculated with use of formula 13.

[Formula 13]

$$ LD_\phi = \frac{d_\phi}{n_\phi} $$

where,

$\varphi$: a direction in which the density is calculated
$LD\varphi$: a density of the contour lines CL in direction $\varphi$
$d_\phi$: a distance from the centroid GP to the intersection $Q_\varphi$
$n_\varphi$: the number of contour lines CL which intersect with a radial line extending in direction $\varphi$ from the centroid GP ($n_\varphi$=3)

(Maximum Density Calculation: S829)

Next, the maximum value LDmax of the densities $LD\varphi$ calculated in the density calculation S828. This value is the intensity gradation of the intensity image $LP_4$.

(Determination of Inspection Type: S83)

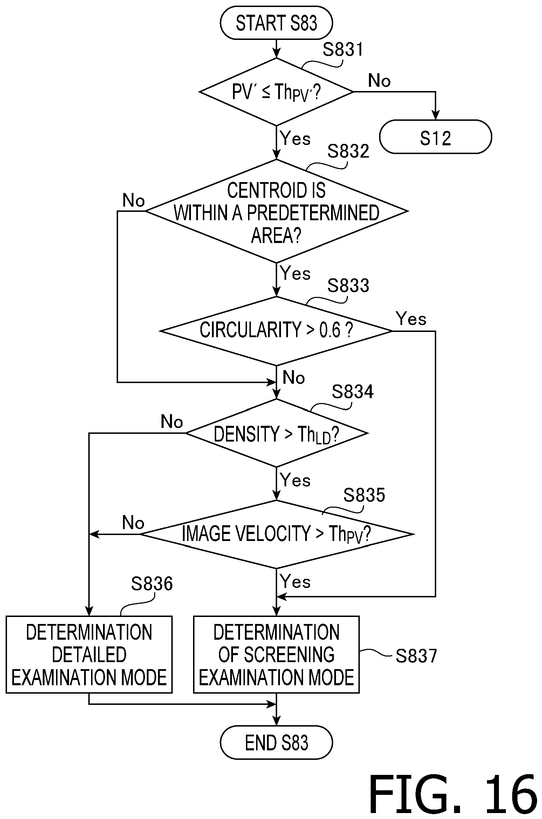

Fig. 16 is a flowchart illustrating a procedure of an inspection type determining process S83.

(Image Velocity Changing Rate Determination: S831)

In the inspection type determining process S83, firstly, an image velocity changing rate determining process S831 to determined whether or not an image velocity changing rate PV' is equal to or less than a predetermined threshold value $Th_{PV'}$ is executed. When a violent movement of the image is occurring such that the image velocity changing rate PV' exceeds the threshold value $Th_{PV'}$, hand shaking occurs on the normal observation image NP, and a marking process (S10, S11) which will be describe later cannot be executed accurately. Further, since the movement of the image is fast, it is difficult for the operator to accurately recognize the marking information. Therefore, when the image velocity changing rate PV' exceeds the threshold value $Th_{PV'}$ (S831: NO), process immediately exits from the inspection type determining process S83, and proceeds to the display image generating process S12 (Fig. 4) without executing the marking process (S10, S11). With this configuration, the normal observation image NP is displayed on the monitor 900 as it is. When the image velocity changing rate PV' is less than the threshold value $Th_{PV'}$ (S831: YES), process proceeds to the next centroid determination S832.

(Centroid Determination: S832)

In Centroid Determination S832, it is determined whether the centroid GP of the lowest gradation area $R_1$ is located within a predetermined central area of the contour line image CP. When the centroid GP is located within the predetermined area (S832: YES), process proceeds to the next circularity determining process S833. When the centroid GP is not within the predetermined area (S832: NO), process proceeds to a density determining process S834 without executing the circularity determining process S833.

(Circularity Determination: S833)

In a circularity determination S833, it is determined whether the circularity $V_{round}$ is greater than a predetermined threshold value (e.g., 0.6) or not. When the circularity $V_{round}$ is greater than the threshold value of 0.6 (S833: YES), the type of inspection is determined to be the screening inspection (S837). When the circularity $V_{round}$ is equal to or less than the threshold value of 0.6 (S833: NO), a density determination S834 is executed subsequently.

(Density Determination: S834)

In the density determination S834, it is judged whether the density LD is greater than a predetermined threshold value $Th_{LD}$. When the density LD is less than the threshold value $Th_{LD}$ (S834: NO), the inspection is determined to be the thorough inspection (S836). When the density LD is equal to or greater than the threshold value $Th_{LD}$ (S834: YES), an image velocity determination S835 is executed subsequently.

(Image Velocity Determination: S835)

In the image velocity determination S835, it is determined whether the image velocity is greater than a predetermined threshold value $Th_{PV}$. When the image velocity PV is greater than the threshold value $Th_{PV}$ (S835: YES), the inspection is determined to be the screening inspection (S837). When the image velocity PV is equal to or less than the threshold value $Th_{PV}$ (S835: NO), the inspection is determined to be the thorough inspection (S836).
Incidentally, the image velocity PV and the image velocity changing rate PV' are parameters regarding movement of the tip of the electronic scope 100 when an inspection is being carried out. Further, the intensity gradient and the circularity $V_{round}$ and a position of the centroid of the lowest gradation area R1 are parameters determined by an attitude of the tip of the electronic scope 100 with respect to the inner wall of the gastrointestinal tract which is an object. That is, the inspection type determination S83 according to the embodiment is to determine the type of the endoscopic inspection based on the movement and attitude of the tip of the electronic scope 100 which is assumed from the endoscopic image.

[Determination of Type of Marking Process: S9]

Next, based on the determination result of the type of inspection in the scene determining process S8, the type of the marking process executed by the marking processing part 226 is determined (S9). When the type of the inspection is determined to be the thorough inspection (S9: YES), a fine marking process S10 is executed. When the type of the inspection is determined to be the screening inspection (S9: NO), the simple marking process S11 is executed.

(Fine Marking: S10)

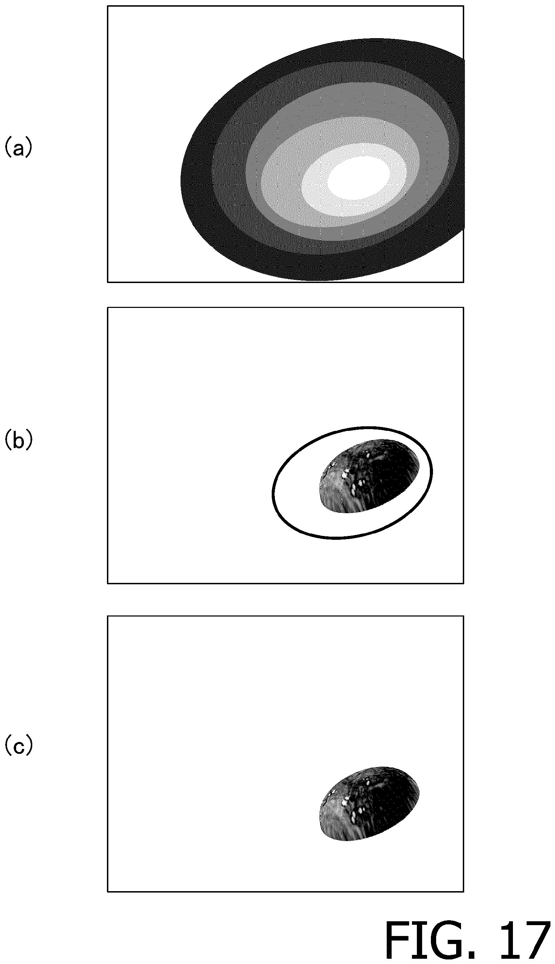

In the fine marking process S10, a color map image CMP in which a distribution of severity degrees in the normal observation image NP is indicated by color is generated as a marking image data to be overlaid on the normal observation image NP. The color map image CMP generated in the fine marking process S10 has display colors Col(x, y), which are determined in accordance with the scores Sc(x, y) of corresponding pixels (x, y) of the normal observation image NP.
In the fine marking process S10, firstly the display color table DCT stored in the memory 229 is referred to and the display colors Col(x, y) to be applied to respective pixels are determined based on the scores Sc(x, y). Then, the color map data CM having the display colors Col(x, y) as pixels values is generated, and stored in the storage area PC of the image memory 227. An example of a color map image CMP generated by the fine marking process S10 is shown in Fig. 17(a). Thereafter, an image composition (i.e., overlay display) to overlay the color map image CMP on the normal observation image NP (or the tone-enhanced image EP) is executed, thereby marking image data M being generated.

It is noted that the display color table DCT is numerical value table defining a relationship between the score Sc and the display colors (i.e., color codes) of the color map image CMP. An example of the display color table DCT is shown in Table 2. Incidentally, regarding the display colors, different colors are set per each eleven steps of scores Sc. To the pixels (x, y) of which scores Sc(x, y) are zero (normal tissues), a value indicating the colorless and transparent (i.e., null value) is assigned. Therefore, the pixels of the normal tissues are not colored by the fine marking process S10. Further, designation of the color to be applied to each pixel (x, y) needs not be limited to designation by RGB, but may be designated by other color expression (e.g., hue and/or saturation). Further, as shown in Fig. 17(a), a colored area may be encircled with an outline having a different display color (e.g., red).

[Table 2]

| SCORE Sc | | 0.0 | 0.1 | 0.2 | 0.3 | 0.4 | 0.5 | 0.6 | 0.7 | 0.8 | 0.9 | 1.0 |
|---|---|---|---|---|---|---|---|---|---|---|---|---|
| DISPLAY COLOR Col | R value | Null | 0 | 0 | 0 | 0 | 0 | 0 | 255 | 255 | 255 | 128 |
| | G value | Null | 0 | 0 | 128 | 128 | 255 | 255 | 255 | 0 | 0 | 128 |
| | B value | Null | 255 | 128 | 128 | 0 | 0 | 255 | 0 | 0 | 255 | 0 |

(Simple Marking: S11)

The simple marking process S11 is a process similar to the fine marking process S10, and a simplified display table DCT (Table 3) is used. Specifically, in the simple marking process S11, to the pixels of which scores Sc is less than a predetermined value (e.g., 0.6) and severity degree is low, a vacant value (i.e., null value) representing colorless and transparent color Col(x, y) is assigned, while to the pixels of which scores Sc is greater than the predetermined value and the severity degree is high, a single display color (e.g., yellow) Col(x, y) is assigned. An example of the marking image MP when the simple marking process S11 is executed is shown in Fig. 17(b).

[Table 3]

| SCORE Sc | | 0.0 - 0.5 | 0.6 - 1.0 |
|---|---|---|---|
| DISPLAY COLOR Col | R value | Pull | 255 |
| | G value | Pull | 255 |
| | B value | Null | 0 |

By employing the simple marking process S11, calculation amount necessary for the process can be largely reduced. Therefore, even for an image of which moving speed is high, the image processing can follow the frame rate, and it becomes possible to apply the marks accurately at the lesion part. Further, since marks of simple configuration (e.g., a single color) is applied at a part where the severity degree is high, the marks are visually well recognizable and the operator can grasp the part of which severity degree is high even in the image of which moving speed is high.

(Modified Embodiment of Simple Marking Process S11)

Fig. 17(c) shows a modified example of the marking image MP generated in the simple marking process S 11. In this modification, the color map image CMP (and the marking image MP), in which a predetermined mark MX (e.g., a mark "▲") is applied at a position where the score Sc exhibits the maximum value in the normal observation image NP, is generated. According to this modification, since it is unnecessary to judge the display color for each pixel, marking can be executed with less processing amount.

(Generation - Output of Display Screen: S12 - S 13)

When the fine marking process S10 or the simple marking process S11 has completed, a display screen generating process S12 is executed subsequently. The display screen generating process S12 is to generate display screen data to display a screen on the monitor 900 using various pieces of image data stored in the image memory 227, and is executed by the display screen generating part 228 of the image processing circuit 220. The display screen generating part 228 is capable of generating plurality of kinds of display image data in accordance with control of the system controller 202. To the display screen data as generated, processing such as a gamma compensation is applied by the output circuit 220b, and then converted into a video signal having a predetermined video format and output to the monitor 900 (outputting process S12).

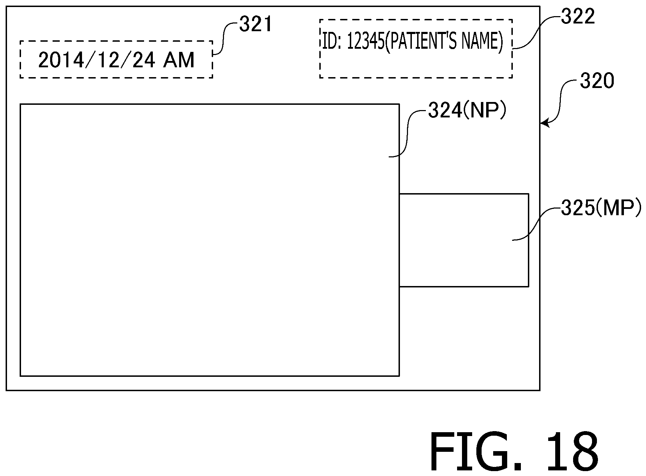

Fig. 18 shows an example of a display screen generated by the display screen generating process S12, and is an analysis mode observation screen 320 which is displayed when the endoscopic observation in the image analyzing mode is carried out. The analysis mode observation screen 320 includes a date/time display area 321 in which photographed date and time are displayed, a basic information display area 322 in which basic information regarding the inspection (e.g., a medical card number, a patient's name, an operator's name), a normal image display area 324 in which the normal observation image NP (or the tone-enhanced image EP) is displayed, and an analysis image display area 325 in which the marking image MP is displayed.

In the display screen generating process S12, the display image generating part 228 retrieves the normal observation image data N (or, retrieves the tone-enhanced image data E from storage area group Pe), and displays the normal observation image NP (or, the tone-enhanced image EP) on the normal image display area 324. Further, the display image generating part 228 retrieves the marking image data M from a storage area group Pm, and displays the marking image MP on the analysis image display area 325. Further, in the date/time display area 321 and the basic information display area 322, information supplied from the system controller 202 is displayed.

The operator carries out the endoscopic observation with watching the analysis mode observation screen 320. Specifically, the operator carries out the endoscopic observation with watching the normal observation image NP (or the tone-enhanced image EP) displayed in the normal image display area 324, with reference to the marking image MP displayed in the analysis image display area 325. By carefully observing particularly carefully a where a marking is applied in the marking image MP, an accurate medical examination can be carried out without overlooking a lesion part.

After completion of the display screen generating process S12 and outputting process S13, it is judged whether the endoscopic observation is to be continued (S14). Until a user operation to instruct end of the endoscopic observation or stoppage of operating the electronic endoscope apparatus 1 is carried out (S14: NO), the processes S1 - S13 are repeated.

It is noted that the above embodiments are examples where the present invention is applied to the electronic endoscope systems. However, the present invention needs not be limited to such a configuration. For example, the present invention can be applied to an image reproducing device configured to reproduce endoscopic observation images photographed by the electronic endoscope apparatus. The present invention can also be applied to an observation image other than the endoscopic images (e.g., observation images taken with ordinary video cameras, or observation images inside a human body during operations).

According to the embodiment, a configuration to determine the type of the endoscopic inspection based on the movement and attitude of the tip of the electronic scope 100, which are assumed from the endoscopic image, is employed. However, the present invention needs not be limited to such a configuration. For example, an insertion shape detecting function to detect a shape and/or position of an insertion part of the endoscope during an inspection (an example of which is disclosed in Japanese Patent Provisional Publication No. 2013-85744) may be provided to the electronic scope, and the type of the endoscopic inspection may be judged based on the movement and the attitude of the tip of the electronic scope which are detection results by the insertion shape detection function.

The foregoing is the description of the illustrative embodiments. The embodiments of the present invention are not limited to those described above, and various modifications can be made within technical philosophy of the present invention. For example, appropriate combinations of illustratively indicated embodiments in the specification are also included in embodiments of the present invention.

**Claims**

1. An image processing apparatus, comprising:

an image data obtaining means configured to obtain color moving image data including multiple pieces of image data capturing biological tissues;
a scene determining means configured to determine a photographic scene based on the color moving image data;
a score calculating means configured to calculate a score indicative of seriousness of lesion of the biological tissues captured in the image represented by the image data for each pixel, based on the image data;
a marking means configured to apply marks indicative of a distribution of the scores on the image,
wherein the marking means is configured to execute:

a detailed marking process to apply the marks indicating the distribution of the scores in detail, and
a simple marking process to apply the marks indicating the distribution of the scores in a manner simpler than the detailed marking process, and

wherein the marking means executes one of the detailed marking process and the simple marking process in accordance with the result of determination of the photographic scene.

2. The image processing apparatus according to claim 1, wherein the scene determining means determines a kind of image an image inspection photographed in the color moving image data.

3. The image processing apparatus according to claim 2,
wherein the scene determining means determines whether the photographic scene is of a screening inspection or a thorough inspection,
wherein the marking means executes:

the simple marking process when the photographic scene is determined to be of the screening inspection, and
the detailed marking process when the photographic scene is determined to be of the thorough inspection.

4. The image processing apparatus according to claim 3,
wherein the scene determining means comprises a moving image analyzing means configured to analyze movement of the image.

5. The image processing apparatus according to claim 4,
wherein the moving image analyzing means:

comprises a velocity field calculating means configured to calculate a velocity field based on continuous multiple pieces of image data; and
determines a kind of the image inspection based on a calculation result of the velocity field.

6. The image processing apparatus according to claim 4 or 5,
wherein the moving image analyzing means comprises an image velocity calculating means configured to calculate a representative value of magnitude of velocity vectors of respective pixels constituting the velocity field and obtain the representative value as an image velocity.

7. The image processing apparatus according to claim 6,
wherein the moving image analyzing means comprises an image velocity change rate calculating means configured to calculate an image velocity change rate which represents a rate of change of the image velocity per unit time.

8. The image processing apparatus according to any one of claims 4 - 7,
wherein the moving image analyzing means comprises a resolution lowering means configured to lower a resolution of the image data.

9. The image processing apparatus according to claim 8,
wherein the scene determining means comprises a brightness image data generating means configured to generate brightness image data of which pixel values are brightness values of the image data.

10. The image processing apparatus according to claim 9,
wherein the scene determining means comprises an image simplifying means configured to simplify a brightness image represented by the brightness image data.

11. The image processing apparatus according to claim 10,
wherein the image simplifying means comprises:

a resolution lowering means configured to lower a resolution of the brightness image;
a blurring means configured to apply a blurring processing to the brightness image of which resolution has been lowered; and
a resolution increasing means configured to increase the resolution of the brightness image to which the blurring processing has been applied to an original resolution.

12. The image processing apparatus according to claim 10 or 11,
wherein the image simplifying means comprises a gradation reducing means configured to reduce gradation of the brightness image data.

**13.** The image processing apparatus according to claim 9,
wherein the scene determining means comprises a contour line image data generating means configured to generate contour line image data representing contour lines of brightness values based on the brightness image.

**14.** The image processing apparatus according to claim 13,
wherein the contour line image data generating means comprises a vector differential calculating means configured to calculate gradient of the brightness image data.

**15.** The image processing apparatus according to claim 13 or 14,
wherein the scene determining means comprises a contour line density calculating means configured to calculate a density of the contour lines, and
wherein the scene determining means determines the photographic scene based on the density of the contour lines.

**16.** The image processing apparatus according to any one of claims 1-15,
wherein the scene determining means comprises a brightness gradient calculating means configured to calculate brightness gradient within the image, and
wherein the scene determining means determines the photographic scene based on the brightness gradient.

**17.** The image processing apparatus according to any one of claims 1 - 16,
wherein the scene determining means comprises a circularity calculating means configured to calculate a circularity of a low brightness area of the image, and
wherein the scene determining means determines the photographic scene based on the circularity.

**18.** The image processing apparatus according to any one of claims 1-17,
wherein the scene determining means has a centroid calculating means configured to calculate a center of gravity of a low brightness area of the image, and
wherein the scene determining means determines the photographic scene based on the centroid.

FIG. 1

FIG. 2

900

220

220b OUTPUT CIRCUIT

228 DISPLAY SCREEN GENERATING PART

227 IMAGE MEMORY

226 MARKING PROCESSING PART

225 SCORE CALCULATING PART

224 LESION DETERMINING PART

223 COLOR SPACE CONVERTING PART

222 EFFECTIVE PIXEL JUDGING PART

221 TE PROCESSING PART

230 SCENE DETERMINING PART

229 MEMORY
FT
ST
HCT
SCT
DCT

220a BASIC PROCESSING PART

108 SOLID STATE IMAGING ELEMENT

112 DRIVING CIRCUIT

110

114 AFE

$P_n$ $P_e$ $P_c$ $P_m$

| NORMAL OBSERVATION IMAGE DATA N | TONE-ENHANCED IMAGE DATA E | COLOR MAP IMAGE DATA CM | MARKING IMAGE DATA M |
|---|---|---|---|

227

# FIG. 3

START

BASIC PROCESSING —— S1

IMAGE ANALYSIS MODE? —— S2

No →

Yes ↓

TE PROCESSING —— S3

EFFECTIVE PIXEL JUDGMENT —— S4

COLOR SPACE CONVERSION —— S5

LESION DETERMINATION —— S6

SCORE CALCULATION —— S7

SCENE DETERMINATION —— S8

THOROUGH INSPECTION MODE ? —— S9

No →

Yes ↓

FINE MARKING —— S10

SIMPLIFIED MARKING —— S11

DISPLAY SCREEN GENERATION —— S12

OUTPUT PROCESS —— S13

OBSERVATION TO BE CONTINUED? —— S14

Yes

No ↓

END

FIG. 4

FIG. 5

START S4

S41

CALCULATE COMPENSATED
LUMINANCE

S42

APPROPRIATE
EXPOSURE?                    No

Yes      S43                              S44

$f(x, y) \leftarrow 1$              $f(x, y) \leftarrow 0$

S45

ALL THE
No      PIXELS HAVE BEEN
PROCESSED?

Yes

END S4

FIG. 6

START S6

S61

f(x, y) ?  →  0

1

S62

DETERMINED TO BE
INFLAMMATION?  →  No

Yes

f(x, y) ← 2  — S63

S64

No  ←  ALL THE
PIXELS HAVE BEEN
PROCESSED?

Yes

END S6

# FIG. 7

FIG. 8

EP 3 263 011 A1

FIG. 9

START S7

S71
LESION PART? — No

Yes

COMPENSATION OF SATURATION — S72

CALCULATE HUE DISTANCE $D_{HUE}$ — S73

DETERMINE HCV(x, y) — S74

CALCULATE SATURATION DISTANCE $D_{SAT}$ — S75

DETERMINE SCV(x, y) — S76

CV(x, y)←HCV(x, y)*SCV(x, y) — S77

Sc(x, y)←CV(x, y) — S78            Sc(x, y) ← 0 — S79

S80
ALL THE PIXELS HAVE BEEN PROCESSED? — No

Yes

END S7

(a)

HUE CORRELATION VALUE HCV

1.0

0.0

$- \leftarrow 0 \rightarrow +$

$D\_HUE$

NON HUE
APPROXIMATION
RANGE $R_{12}$

HUE
APPROXIMATION
$-30°$ RANGE $R_{11}$ $+30°$

NON HUE
APPROXIMATION
RANGE $R_{12}$

(b)

SATURATION CORRELATION VALUE SCV

1.0

0.0

$- \leftarrow 0 \rightarrow +$

$D\_SAT$

NON SATURATION
APPROXIMATION
RANGE $R_{23}$

SATURATION
APPROXIMATION
RANGE $R_{22}$

SATURATION
COINCIDENCE
RANGE $R_{21}$

# FIG. 10

(a)

(b)

(c)

FIG. 11

START S8

ANALYSIS OF MOTIN PICTURE — S81

CALCULATION OF INTENSITY GRADIENT — S82

DETERMINATION OF INSPECTION TYPE — S83

END S8

# FIG. 12

START S81

MAKE LOW-RESOLUTION DATA — S811

CALCULATE VELOCITY FIELD — S812

CALCULATE AVERAGE VELOCITY — S813

CALCULATE VELOCITY CHANGING RATE — S814

END S81

# FIG. 13

START S82

CALCULATE INTENSITY INDEX ~ S820

MAKE LOW-RESOLUTION DATA ~ S821

BLURRING ~ S822

MAKE HIGH-RESOLUTION DATA ~ S823

MAKE LOW-GRADATION DATA ~ S824

VECTOR DIFFERENTIAL OPERATION ~ S825

CIRCULARITY CALCULATION ~ S826

CENTROID CALCULATION ~ S827

CONTOUR LINE DENSITY CALCULATION ~ S828

MAXIMUM DENSITY CALCULATION ~ S829

END S82

# FIG. 14

FIG. 15

FIG. 16

(a)

(b)

(c)

FIG. 17

2014/12/24 AM

321

ID: 12345(PATIENT'S NAME)

322

320

324(NP)

325(MP)

FIG. 18

## INTERNATIONAL SEARCH REPORT

| International application No. |
| --- |
| PCT/JP2016/055150 |

A.  CLASSIFICATION OF SUBJECT MATTER
*A61B1/04*(2006.01)i, *A61B1/00*(2006.01)i

According to International Patent Classification (IPC) or to both national classification and IPC

B.  FIELDS SEARCHED

Minimum documentation searched (classification system followed by classification symbols)
A61B1/00-1/32

Documentation searched other than minimum documentation to the extent that such documents are included in the fields searched
Jitsuyo Shinan Koho        1922–1996   Jitsuyo Shinan Toroku Koho   1996–2016
Kokai Jitsuyo Shinan Koho   1971–2016   Toroku Jitsuyo Shinan Koho   1994–2016

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| A | JP 2014-213094 A  (Hoya Corp., Kyoto University), 17 November 2014 (17.11.2014), paragraph [0032]; fig. 2 & US 2014/0320620 A1 paragraph [0036]; fig. 2 | 1-18 |
| A | JP 2012-130429 A  (Fujifilm Corp.), 12 July 2012 (12.07.2012), fig. 3 & US 2012/0157775 A1 fig. 3 & EP 2465433 A1 | 1-18 |

☐ Further documents are listed in the continuation of Box C.          ☐ See patent family annex.

| *    Special categories of cited documents: |
| --- |
| "A"  document defining the general state of the art which is not considered    to be of particular relevance |
| "E"  earlier application or patent but published on or after the international filing date |
| "L"  document which may throw doubts on priority claim(s) or which is cited to establish the publication date of another citation or other special reason (as specified) |
| "O"  document referring to an oral disclosure, use, exhibition or other means |
| "P"  document published prior to the international filing date but later than the priority date claimed |

| "T"  later document published after the international filing date or priority date and not in conflict with the application but cited to understand the principle or theory underlying the invention |
| --- |
| "X"  document of particular relevance; the claimed invention cannot be considered novel or cannot be considered to involve an inventive step when the document is taken alone |
| "Y"  document of particular relevance; the claimed invention cannot be considered to involve an inventive step when the document is combined with one or more other such documents, such combination being obvious to a person skilled in the art |
| "&"  document member of the same patent family |

| Date of the actual completion of the international search | Date of mailing of the international search report |
| --- | --- |
| 09 May 2016 (09.05.16) | 17 May 2016 (17.05.16) |

| Name and mailing address of the ISA/ Japan Patent Office 3-4-3,Kasumigaseki,Chiyoda-ku, Tokyo 100-8915,Japan | Authorized officer Telephone No. |
| --- | --- |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014018332 A

- JP 2013085744 A